# EUROPÄISCHE PATENTSCHRIFT

(11) **EP 3 419 433 B1**
(45) Veröffentlichungstag und Bekanntmachung des Hinweises auf die Patenterteilung: **02.10.2019**
(21) Anmeldenummer: 17706755.0
(22) Anmeldetag: 22.02.2017
(51) Int. Cl.: A23G 3/36, A23G 4/06, A61K 9/68, A23P 10/30, A23L 27/30, A61K 8/60, A61K 8/81, A61K 8/11, A61Q 11/00

(54) **VERKAPSELTE SÜSSSTOFFE UND VERFAHREN ZU IHRER HERSTELLUNG**
ENCAPSULATED SWEETENERS AND METHOD FOR PRODUCING SAME
ÉDULCORANTS EN CAPSULES ET PROCÉDÉ DE FABRICATION DE CES ÉDULCORANTS

(30) Priorität: 25.02.2016 DE 102016203008
(43) Veröffentlichungstag der Anmeldung: 02.01.2019
(73) Patentinhaber: Wacker Chemie AG, 81737 München (DE)
(72) Erfinder: WIMMER, Thomas, 84489 Burghausen (DE)
(86) Internationale Anmeldenummer: PCT/EP2017/054035
(87) Internationale Veröffentlichungsnummer: WO 2017/144520

(56) Entgegenhaltungen:
- WO-A1-2008/119674
- US-A- 5 165 944
- US-A1- 2009 246 327

## Beschreibung

Die Erfindung betrifft verkapselte Süßstoffe sowie Verfahren zu deren Herstellung.

In zahnfreundlichen Kaugummis werden die süßen Inhaltsstoffe Zucker und Glucosesirup häufig durch Polyole in kristalliner Form und in Form von konzentrierten Lösungen, z.B. Maltitsirup, ersetzt. Wichtige Zuckeraustauschstoffe sind die Polyole wie z.B. Sorbitol, Maltitol, Isomalt, Mannitol, Xylitol, Erythritol, Palatinose oder Lactitol.

Da die Süßkraft der Polyole meist geringer ist als die von Zucker werden in der Regel in zuckerfreien Kaugummis zusätzlich Süßstoffe eingesetzt. Als Süßstoffe sollen Verbindungen verstanden werden, die eine im Vergleich zu Zucker vielfach höhere Süßkraft aufweisen und keinen oder nur einen vernachlässigbaren Nährwert haben. Zu den Süßstoffen gehören z.B. Aspartam, Acesulfam K, Aspartam-Acesulfam-Salz, Sucralose, Cyclamate, Neohesperidin, Saccharin, Stevia, Alitam, Neotam, und Thaumatin. Die Süßstoffe werden dabei als Einzelstoffe oder als synergistische Mischungen eingesetzt.

Ein Nachteil von Süßstoffen in Kaugummi ist, dass die Süße relativ schnell durch den Kauprozess verloren geht. Eine bekannte Möglichkeit diesen Nachteil zu verringern ist es, die Freisetzung von Süßstoffen durch Verkapselung zu verzögern.

So beschreibt z.B. US4384004 die Verkapselung von Aspartam mit Ethylcellulose, Hydroxypropylcellulose oder Polyvinyl-pyrrolidon. Die Herstellung von mit Wachs überzogenem Acesulfam K und die Verwendung in Kaugummi werden in US4885175 beschrieben. Auch die Verkapselung mit Polyvinylacetat ist durch zahlreiche Patente bekannt. In US5169658 wird die Verkapselung von Sucralose mit in der Wärme erweichtem Polyvinylacetat (Molmassse = 32.000 g/mol) in einem Doppel-Z-Kneter beschrieben. Die verzögerte Freisetzung in Kaugummi wird sensorisch und analytisch nachgewiesen. In Beispiel 12 des US4997659 wird pulverförmiges Alitame und Polyvinylacetat in einem Schmelzspinnverfahren extrudiert. US5165944 offenbart ein Verfahren zur Verkapselung von Acesulfam-K, Aspartam, Saccharin mit Polyvinylacetat (Molmasse 50.000 - 80.000 g/mol)in einem Extruder. Die erhaltenen Spinnfäden werden zerkleinert und bewirken in Kaugummi einen länger anhaltenden Geschmack.

Die Verkapselung von Aspartam und Saccharin in einer geschmolzenen Mischung aus hochmolekularem Polyvinylacetat und Glycerinmonostearat wird beschrieben in US4711784.

Die Herstellung eines verkapselten Aspartam mit Polyvinylacetat und Magnesiumstearat in einem Extrusionsprozess wird beschrieben in US7244454. Eine ausführliche Beschreibung der Herstellung eines granulierten, verkapselten Süßstoffes (Acesulfam-K) mit Polyvinylacetat, teilhydriertem Sojaöl und Glycerinmonostearat findet sich in US5000965. Eine Abhängigkeit der Freisetzung der Süße von der Zugfestigkeit einer Mischung aus hochmolekularem Polyvinylacetat, hydriertem Öl, Glycerinmonostearat und den Süßstoffen Aspartam und Acesulfam-K wird offenbart in US8828423.

Die Aufgabe der Erfindung ist es, eine neue Komposition von verkapselten Süßstoffen bereitzustellen.

Die Aufgabe wird gelöst durch eine Zusammensetzung gemäß Anspruch 1.

Gegebenenfalls enthält die Zusammensetzung ferner 0 - 10 Gew.% Additive aus der Gruppe Emulgatoren und Fette.

Vorzugsweise besteht die Zusammensetzung zu 10 - 30 Gew.-% aus Süßstoffen und zu 70-90 Gew.-% aus Vinylacetat-Vinyllaurat-Copolymeren und 0 - 10 Gew.% Additiven aus der Gruppe Emulgatoren und Fette.

Als Süßstoffe sind offenbart Aspartam, Acesulfam K, Aspartam-Acesulfam-Salz, Sucralose, Cyclamate, Neohesperidin, Saccharin, Stevia, Alitam, Neotam, und Thaumatin. Süßstoffe können allein oder in synergistischen Mischungen eingesetzt werden. Mischungen verschiedener Süßstoffe haben bekannterweise einen positiven Effekt auf Süßkraft und eine Verbesserung im Geschmacksprofil in der Anwendung. In der Erfindung verwendete Süßstoffe sind Aspartam, Acesulfam K und Sucralose und Mischungen dieser Süßstoffe.

Die erfindungsgemäß eingesetzten Vinylacetat-Vinyllaurat-Copolymere haben ein Monomerverhältnis im Polymer von 4 - 15 Gew.% Vinyllaurat und entsprechend 85- 96 Gew.% Vinylacetat.

Die gewichtsmittlere Molmasse der Vinylacetat-Vinyllaurat-Copolymere liegt bevorzugt bei 10.000 - 250.000 g/mol, besonders bevorzugt bei 20.000 bis 150.000 g/mol. Die gewichtsmittlere Molmasse Mw wurde bestimmt mittels Size Exclusion Chromatographie (SEC) gegen Polystyrol-Standard, in THF, bei 40 °C, Flow rate 1,2 ml/min.

Als geeignete Emulgatoren können erfindungsgemäß Glycerinmonostearat, acetylierte Glycerinester von Fettsäuren, Fettsäureester der Saccharose, Diacetylweinsäureester von Monoglyceriden, Milchsäure- oder Citronensäureester von Monoglyceriden oder Lecithin verwendet werden. Bevorzugt sind Glycerinmonostearat und acetylierte Glycerinester von Fettsäuren
Geeignete Fette sind ungehärtete, teilhydrierte und voll hydrierte tierische Fette und pflanzliche Öle wie z.B. Rindertalg, Soyaöl, Erdnußöl, Baumwollsamenöl, Palmöl, Palmkernöl, Rapsöl oder Sonnenblumenöl.

Die erfindungsgemäßen Zusammensetzungen können hergestellt werden durch Aufschmelzen des Copolymers bei 70 - 140 °C und Vermischen mit dem Süßstoff und gegebenenfalls des Additives.

Der Süßstoff kann dabei gleichzeitig oder später zugegeben werden. Das Mischen kann kontinuierlich oder batchweise durchgeführt werden.

Zum Vermischen kann ein beheizbarer Doppel-Z-Kneter, ein Extruder, ein Rührkessel oder ein Planetenmischer verwendet werden.

Nach dem Vermischen wird die Masse abgekühlt und zerkleinert. Hierbei können exemplarisch Verfahren wie z.B. Granulieren, Schneidgranulieren, Unterwasserschneidgranulieren, Brechen, Mahlen oder kryogenes Mahlen verwendet werden. Bei Bedarf können die zerkleinerten Produkte durch Sieben oder Windsichten klassiert werden.

Die erfindungsgemäßen Zusammensetzungen lassen sich in verschiedenen Produkten wie beispielsweise Süßwaren, Medizinprodukte wie z.B. Zahnhaftcremes, pharmazeutische Zubereitungen, sowie kosmetische Produkte, z.B. Zahnpasta, einarbeiten.

Besonders geeignet ist die Verwendung in Kaugummis, speziell zuckerfreie Kaugummis. Zuckerfreie Kaugummis bestehen in der Regel aus Gumbase, Polyolen, Aromen, Süßstoffen und weiteren Additiven wie z.B. Farbstoffen, Antioxidantien, Emulgatoren, Weichmacher und Feuchthaltemitteln. Als Polyole finden Sorbitol, Maltitol, Isomalt, Mannitol, Xylitol, Erythritol, Palatinose oder Lactitol oder eine Mischung zweier oder mehrerer dieser Substanzen, die teilweise auch als Sirupe erhältlich sind, Verwendung.

Die erfindungsgemäßen Zusammensetzungen bewirken dabei eine verzögerte Freisetzung der Süße und man erreicht damit einen länger anhaltenden Geschmack des Kaugummis.

Die folgenden Beispiele dienen der weiteren Erläuterung der Erfindung.

### Beispiel 1: Herstellung eines Vinylacetat-Vinyllaurat-Copolymers

In einem Rührkessel wurden 3 kg Isopropanol zusammen mit 2 kg Vinyllaurat, 38 kg Vinylacetat und 8 g t-Butylperoxo-2-ethylhexanoat vorgelegt und die Polymerisation mittels Aufheizen der Vorlage auf 72°C gestartet. Zum Start wurden 8 g t-Butylperoxo-2-ethylhexanoat zugegeben und während der Polymerisation 100 g t-Butylperoxo-2-ethylhexanoat in 1200 g Isopropanol innerhalb von 5 h zudosiert. Nach einer weiteren Stunde wurde die Temperatur auf 120 °C erhöht. Anschließend wurde der Kessel evakuiert und dabei Lösungsmittel und Restmonomere abdestilliert. Die Schmelze wurde abgelassen und abgekühlt. Man erhielt ein klares Produkt mit einer Monomerzusammensetzung (Gew.%) von 95% Vinylacetat und 5% Vinyllaurat. Die Glasübergangstemperatur wurde mit Differential Scanning Calorimetry (DSC) zu 37 °C bestimmt. Die gewichtmittlere Molmasse wurde per SEC (Size Exclusion Chrommatography) gemessen mit 105.000 g/mol.

### Beispiel 2: Herstellung eines Vinylacetat-Vinyllaurat-Copolymers

In einem Rührkessel wurden 4 kg Isopropanol zusammen mit 4,8 kg Vinyllaurat, 35,2 kg Vinylacetat und 10 g t-Butylperoxo-2-ethylhexanoat vorgelegt und die Polymerisation mittels Aufheizen der Vorlage auf 72°C gestartet. Zum Start wurden 8 g t-Butylperoxo-2-ethylhexanoat zugegeben und während der Polymerisation 136 g t-Butylperoxo-2-ethylhexanoat in 1500 g Isopropanol innerhalb von 5 h zudosiert. Nach einer weiteren Stunde wurde die Temperatur auf 120 °C erhöht. Anschließend wurde der Kessel evakuiert und dabei Lösungsmittel und Restmonomere abdestilliert. Die Schmelze wurde abgelassen und abgekühlt. Man erhielt ein klares Produkt mit einer Monomerzusammensetzung (Gew.%) von 88% Vinylacetat und 12% Vinyllaurat. Die Glasübergangstemperatur wurde mit DSC zu 32 °C bestimmt. Die gewichtmittlere Molmasse wurde per SEC gemessen mit 46.000 g/mol.

### Beispiel 3: Herstellung eines verkapselten Aspartams

700 g eines Vinylacetat-Vinyllaurat-Copolymers aus Beispiel 1 wurden in einem Doppel-Z-Kneter bei 105°C aufgeschmolzen. Anschließend wurde 300 g feinkristallines Aspartam zugegeben, die Masse noch 10 Minuten bei 105°C geknetet und aus dem Kneter entleert. Nach dem Erkalten wurde die Masse in einer Mühle zerkleinert. Die Aspartam Konzentration wurde mittels HPLC zu 30% bestimmt.

### Beispiel 4:Herstellung von verkapselter Sucralose

750 g eines Vinylacetat-Vinyllaurat-Copolymers aus Beispiel 2 und 50 g von Glycerinmonostearat wurden in einem Doppel-Z-Kneter bei 95°C aufgeschmolzen. Anschließend wurden 200 g pulverisierte Sucralose zugegeben, die Masse noch 10 Minuten bei 95°C geknetet und aus dem Kneter entleert. Nach dem Erkalten wurde die Masse in einer Mühle zerkleinert. Die Sucralose Konzentration wurde mittels HPLC zu 20% bestimmt.

### Beispiel 5:Herstellung von verkapselter Acesulfam K

700 g eines Vinylacetat-Vinyllaurat-Copolymers aus Beispiel 1 wurden mit 30 g Glycerinmonostearat und 50 g gehärtetes Palmfett in einem Doppel-Z-Kneter bei 100°C aufgeschmolzen. Anschließend wurden 220 g Acesulfam K zugegeben, die Masse noch 5 Minuten bei 100°C geknetet und aus dem Kneter entleert. Nach dem Erkalten wurde die Masse in einer Mühle zerkleinert. Die Acesulfam K-konzentration wurde mittels HPLC zu 22% bestimmt.

### Beispiel 6: Herstellung eines zuckerfreien Kaugummis (Vergleichsbeispiel)

Aus 300 g einer Gumbase (Valencia T-PL / CAFOSA), 450 g Sorbitol, 108 g Xylitol, 50 g Mannitol, 75 g Maltitol-Sirup (Lycasin® 80/55), 15 g Minzöl und 2,0 g Aspartam wird in einem Doppel-Z-Kneter bei 54°C eine Kaugummimasse hergestellt. Die Masse wurde abgekühlt, auf ca. 1 mm Dicke ausgerollt und in Streifen zu je 2 g geschnitten.

### Beispiel 7: Herstellung eines zuckerfreien Kaugummis (erfindungsgemäß)

Die Herstellung erfolgt wie in Beispiel 6 beschrieben, nur dass die 2 g Aspartam durch 6,67 g verkapseltes Aspartam (aus Beispiel 3) ersetzt wurden.

### Beispiel 8:

Die Kaugummis aus Beispiel 6 und 7 wurden von einzelnen Personen 2, 5, 10, 15 und 20 Minuten gekaut. Die verbleibende Kaumasse wurde mittels HPLC auf den verbliebenen Aspartam Gehalt analysiert.

Die nachstehende Tabelle zeigt klar die verzögerte Freisetzung des Süßstoffs in der erfindungsgemäßen Formulierung.

| Kauzeit | Wiederfindung Aspartam (Bsp. 6) | Wiederfindung Aspartam (Bsp. 7) |
|---|---|---|
| 0 Minuten | 100% | 100% |
| 2 Minuten | 93% | 98% |
| 5 Minuten | 81% | 95% |
| 10 Minuten | 71% | 81% |
| 15 Minuten | 50% | 79% |
| 20 Minuten | 41% | 69% |

## Patentansprüche

1. Zusammensetzung von verkapselten Süßstoffen enthaltend 1-40 Gew.-% Süßstoffe, wobei die Süßstoffe ausgewählt sind aus der Gruppe Aspartam, Acesulfam K und Sucralose und Mischungen dieser Süßstoffe, sowie 60 - 99 Gew.-% Vinylacetat-Vinyllaurat-Copolymere wobei ein Monomerverhältnis im Copolymer von 4 - 15 Gew.% Vinyllaurat und 85 - 96 Gew.% Vinylacetat vorliegt.

2. Zusammensetzung gemäß Anspruch 1 enthaltend zusätzlich 0-10 Gew.% Additive aus der Gruppe Emulgatoren und Fette.

3. Zusammensetzung gemäß Anspruch 1 oder 2, bestehend aus 10 - 30 Gew.-% Süßstoffen und 70 - 90 Gew.-% Vinylacetat-Vinyllaurat-Copolymere und 0 - 10 Gew.% Additive aus der Gruppe Emulgatoren und Fette.

4. Zusammensetzung gemäß einem der Ansprüche 1 bis 3, **dadurch gekennzeichnet, dass** die gewichtsmittlere Molmasse der Vinylacetat-Vinyllaurat-Copolymere 10.000 - 250.000 g/mol beträgt.

5. Zusammensetzung gemäß einem der Ansprüche 2 bis 4, **dadurch gekennzeichnet, dass** der Emulgator ausgewählt ist aus der Gruppe Glycerinmonostearat, acetylierte Glycerinester von Fettsäuren, Fettsäureester der Saccharose, Diacetylweinsäureester von Monoglyceriden, Milchsäure- oder Citronensäureester von Monoglyceriden und Lecithin.

6. Zusammensetzung gemäß einem der Ansprüche 2 bis 5, **dadurch gekennzeichnet, dass** das Fett ausgewählt ist aus der Gruppe ungehärtete, teilhydrierte und voll hydrierte tierische Fette und pflanzliche Öle.

7. Verfahren zur Herstellung einer Zusammensetzung gemäß einem oder mehreren der Ansprüche 1 bis 6, **dadurch gekennzeichnet, dass** das Copolymer bei 70 - 140 °C aufgeschmolzen wird und mit dem Süßstoff und gegebenenfalls den Additiven vermischt, abgekühlt und zerkleinert wird.

8. Verwendung einer Zusammensetzung gemäß einem oder mehreren der Ansprüche 1 bis 6 zur Einarbeitung in Süßwaren, Medizinprodukte oder Kaugummis.

## Claims

1. Composition of encapsulated sweeteners comprising 1-40 wt% of sweeteners, wherein the sweeteners are selected from the group consisting of aspartame, acesulfame K and sucralose and mixtures of these sweeteners, and also 60-99 wt% of vinyl acetate-vinyl laurate copolymers, wherein the copolymer has a monomer ratio of 4-15 wt% of vinyl laurate and 85-96 wt% of vinyl acetate.

2. Composition according to Claim 1, additionally comprising 0-10 wt% of additives from the group of emulsifiers and fats.

3. Composition according to Claim 1 or 2, consisting of 10-30 wt% of sweeteners and 70-90 wt% of vinyl acetate-vinyl laurate copolymers and 0-10 wt% of additives from the group of emulsifiers and fats.

4. Composition according to one of Claims 1 to 3, **characterized in that** the weight-average molar mass of the vinyl acetate-vinyl laurate copolymers is 10 000 - 250 000 g/mol.

5. Composition according to one of Claims 2 to 4, **characterized in that** the emulsifier is selected from the group consisting of glycerol monostearate, acetylated glycerol esters of fatty acids, fatty acid esters of sucrose, diacetyltartaric acid esters of monoglycerides, lactic acid esters or citric acid esters of monoglycerides and lecithin.

6. Composition according to one of Claims 2 to 5, **characterized in that** the fat is selected from the group consisting of unhydrogenated, partially hydrogenated and fully hydrogenated animal fats and vegetable oils.

7. Method for preparing a composition according to one or more of Claims 1 to 6, **characterized in that** the copolymer is melted at 70 - 140°C and mixed with the sweetener and optionally the additives, cooled and comminuted.

8. Use of a composition according to one or more of Claims 1 to 6 for incorporation into confectionery products, medicinal products or chewing gums.

## Revendications

1. Composition d'édulcorants encapsulés contenant 1 à 40 % en poids d'édulcorants, les édulcorants étant choisis dans le groupe constitué par l'aspartame, l'acésulfame K et le sucralose et les mélanges de ces édulcorants, ainsi que 60 à 99 % en poids de copolymères d'acétate de vinyle-laurate de vinyle, un rapport entre les monomères dans le copolymère de 4 à 15 % en poids de laurate de vinyle et 85 à 96 % en poids d'acétate de vinyle étant présent.

2. Composition selon la revendication 1, contenant en outre 0 à 10 % en poids d'additifs du groupe constitué par les émulsifiants et les matières grasses.

3. Composition selon la revendication 1 ou 2, constituée par 10 à 30 % en poids d'édulcorants et 70 à 90 % en poids de copolymères d'acétate de vinyle-laurate de vinyle, et 0 à 10 % en poids d'additifs du groupe constitué par les émulsifiants et les matières grasses.

4. Composition selon l'une quelconque des revendications 1 à 3, **caractérisée en ce que** la masse molaire moyenne en poids des copolymères d'acétate de vinyle-laurate de vinyle est de 10 000 à 250 000 g/mol.

5. Composition selon l'une quelconque des revendications 2 à 4, **caractérisée en ce que** l'émulsifiant est choisi dans le groupe constitué par le monostéarate de glycérine, les esters de glycérine acétylés d'acides gras, les esters d'acides gras de saccharose, les esters de l'acide diacétyltartrique de monoglycérides, les esters de l'acide lactique ou de l'acide citrique de monoglycérides et la lécithine.

6. Composition selon l'une quelconque des revendications 2 à 5, **caractérisée en ce que** la matière grasse est choisie dans le groupe constitué par les matières grasses animales et les huiles végétales non durcies, partiellement hydrogénées et entièrement hydrogénées.

7. Procédé de fabrication d'une composition selon une ou plusieurs des revendications 1 à 6, **caractérisé en ce que** le copolymère est fondu à une température de 70 à 140 °C, et mélangé avec l'édulcorant et éventuellement les additifs, refroidi et broyé.

8. Utilisation d'une composition selon une ou plusieurs des revendications 1 à 6 pour l'incorporation dans des confiseries, des produits médicaux ou des chewing-gums.
